(19) Europäisches Patentamt
European Patent Office
Office européen des brevets

(11) Veröffentlichungsnummer : **0 135 469**
**B1**

(12) # EUROPÄISCHE PATENTSCHRIFT

(45) Veröffentlichungstag der Patentschrift : **16.06.87**

(21) Anmeldenummer : **84810308.1**

(22) Anmeldetag : **22.06.84**

(51) Int. Cl.⁴ : **C 07 D401/02**, C 07 D403/02, C 07 D413/02, B 41 M 5/16, B 41 M 5/18, B 41 M 5/22

(54) **Chromogene Chinazolinverbindungen.**

(30) Priorität : **28.06.83 CH 3522/83**

(43) Veröffentlichungstag der Anmeldung : **27.03.85 Patentblatt 85/13**

(45) Bekanntmachung des Hinweises auf die Patenterteilung : **16.06.87 Patentblatt 87/25**

(84) Benannte Vertragsstaaten : **BE CH DE FR GB IT LI**

(56) Entgegenhaltungen :
**DE-A- 2 345 064**
**GB-A- 1 131 126**

(73) Patentinhaber : **CIBA-GEIGY AG**
**Klybeckstrasse 141**
**CH-4002 Basel (CH)**

(72) Erfinder : **Zink, Rudolf**
***Alemannenstrasse 2***
**CH-4106 Therwil (CH)**
Erfinder : **Fletcher, Ian John, Dr.**
**Bürgenstal 27**
**CH-4312 Magden (CH)**

**EP 0 135 469 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft chromogene Chinazolinverbindungen, Verfahren zu ihrer Herstellung und ihre Verwendung als Farbbildner in druckempfindlichen oder wärmeempfindlichen Aufzeichnungsmaterialien.

Die neuen chromogenen Chinazolinverbindungen entsprechen der allgemeinen Formel

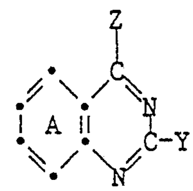

(1)

worin

Y den Rest einer hydrierten stickstoffhaltigen heterocyclischen Verbindung, die über einen in ihr enthaltenden ankondensierten Benzolkern mit dem Chinazolinbestandteil verbunden und unsubstituiert oder substituiert ist und

Z Wasserstoff, R, $-OR_1$, $-SR_1$ oder $-NR_2R_3$ bedeuten, wobei

R und $R_1$ je unsubstituiertes oder durch Cyano oder Niederalkoxy substituiertes Alkyl mit höchstens 12 Kohlenstoffatomen, Cycloalkyl, Phenyl, Naphthyl, Diphenyl, Benzyl oder Phenylethyl, die jeweils unsubstituiert oder durch Halogen, Cyano, Nitro, Niederalkyl, Niederalkoxy, Niederalkoxycarbonyl oder Niederalkylcarbonyl substituiert sind, einen fünf- oder sechsgliedrigen, heterocyclischen Rest aromatischen Charakters, der unsubstituiert oder durch Halogen, Cyano, Niederalkyl, Niederalkoxy oder Niederalkoxycarbonyl substituiert ist und $R_1$ auch Halogenalkyl mit 2 bis 6 Kohlenstoffatomen sein kann,

$R_2$ und $R_3$ unabhängig voneinander, Wasserstoff, unsubstituiertes oder durch Halogen, Hydroxy, Cyano oder Niederalkoxy substituiertes Alkyl mit höchstens 12 Kohlenstoffatomen, Cycloalkyl, Phenyl, Benzyl oder durch Halogen, Nitro, Cyano, Niederalkyl, Niederalkoxy oder Niederalkoxycarbonyl substituiertes Phenyl oder Benzyl und $R_2$ auch Acyl mit 1 bis 12 Kohlenstoffatomen, oder

$R_2$ und $R_3$ zusammen mit dem sie verbindenden Stickstoffatom einen fünf- oder sechsgliedrigen, gesättigten heterocyclischen Rest bedeuten, und der Ring

A unsubstituiert oder durch Halogen, Cyano, Nitro, Niederalkyl, Niederalkoxy oder Niederalkoxycarbonyl substituiert sein kann, wobei der Ausdruck « Nieder » sich auf solche Gruppen mit 1 bis 5 Kohlenstoffatomen bezieht.

Niederalkyl und Niederalkoxy stellen bei der Definition der Reste der Chinazolinverbindungen solche Gruppen oder Gruppenbestandteile dar, die 1 bis 5, insbesondere 1 bis 3 Kohlenstoffatome aufweisen, wie z. B. Methyl, Ethyl, n-Propyl, Isopropyl, n-Butyl, sek.-Butyl, tert.-Butyl oder Amyl bzw. Methoxy, Ethoxy oder Isopropoxy.

« Acyl » ist besonders Formyl, Niederalkylcarbonyl, wie z. B. Acetyl oder Propionyl, oder Benzoyl. Weitere Acylreste sind Niederalkylsulfonyl, wie z. B. Methylsulfonyl oder Ethylsulfonyl sowie Phenylsulfony. Phenyl, Benzoyl und Phenylsulfonyl können durch Halogen, Methyl, Methoxy oder Ethoxy substituiert sein.

Halogen bedeutet beispielsweise Fluor, Brom oder vorzugsweise Chlor.

Stellen die Substituenten R, $R_1$, $R_2$ und $R_3$ Alkylgruppen dar, so können sie geradkettig oder verzweigt sein. Beispiele für solche Alkylreste sind Methyl, Ethyl, n-Propyl, Isopropyl, n-Butyl, sek.-Butyl, Amyl, n-Hexyl, 2-Ethyl-hexyl, Isooctyl, n-Octyl, Decyl oder n-Dodecyl.

Sind die Alkylreste in R, $R_1$, $R_2$ und $R_3$ substituiert, so handelt es sich vor allem um Cyanoalkyl oder Alkoxyalkyl, die jeweils vorzugsweise insgesamt 2 bis 6 Kohlenstoffatome aufweisen, wie z. B. β-Cyanoethyl, β-Methoxyethyl oder β-Ethoxyethyl. Als Halogenalkyl kann $R_1$ beispielsweise γ-Chlorpropyl oder vorzugsweise β-Chloroethyl sein.

Der Acylrest in $R_2$ ist beispielsweise Formyl, Niederalkylcarbonyl oder Benzoyl, vorzugsweise jedoch Acetyl oder Propionyl. Benzoyl kann im Benzolring durch Halogen, Methyl oder Methoxy substituiert sein.

Beispiele für Cycloalkyl in der Bedeutung von R, $R_1$, $R_2$ und $R_3$ sind Cyclopentyl oder vorzugsweise Cyclohexyl.

Bevorzugte Substituenten in der Benzyl- und Phenylgruppe der R-, $R_1$-, $R_2$- und $R_3$-Reste sind z. B. Halogen, Cyano, Methyl, Methoxy oder Carbomethoxy. Beispiele für derartige araliphatische bzw. aromatische Reste sind Methylbenzyl, Chlorbenzyl, Cyanophenyl, Tolyl, Xylyl, Chlorphenyl, Methoxyphenyl oder Carbomethoxyphenyl.

Als heterocyclischer Rest stellen R und $R_1$ einen fünf- oder sechsgliedrigen, vorzugsweise Sauerstoff, Schwefel oder Stickstoff aufweisenden Heterocyclus von aromatischen Charakter dar. Beispiele derartiger Heterocyclen sind Thienyl, Furyl, Pyrrolyl, Pyrazolyl, Imidazolyl oder vorzugsweise Pyridyl. Diese heterocyclischen Reste können substituiert sein, insbesondere durch Halogen, Cyano, Niederalkyl, Niederalkoxy oder Niederalkoxycarbonyl. Die bevorzugten heterocyclischen Reste in der

Bedeutung von R und $R_1$ sind 2-Furyl, 2-Thienyl und besonders 2-, 3- oder 4-Pyridyl.

Wenn $R_2$ und $R_3$ zusammen mit dem gemeinsamen Stickstoffatom einen gesättigten heterocyclischen Rest darstellen, so ist dieser beispielsweise Pyrrolidino, Piperidino, Pipecolino, Morpholino, Thiomorpholino oder Piperazino, wie z. B. N-Methylpiperazino. Bevorzugte gesättigte heterocyclische Reste für $NR_2R_3$ sind Pyrrolidino, Piperidino oder Morpholino.

In Formel (1) ist Z vorzugsweise der Rest —$OR_1$.

Als hydrierte stickstoffhaltige heterocyclische Verbindungen für den Rest Y kommen beispielsweise N-unsubstituierte oder N-substituierte Indoline, Tetrahydrocarbazole, Dihydro- oder Tetrahydrochinoline, Dibenzylimide oder Benzomorpholine in Betracht. Dabei ist Y über den ankondensierten Benzolring der genannten Heterocyclen mit dem Chinazolinteil verbunden. Bevorzugt sind für Y die Indolin-, Tetrahydrochinolin- und Benzomorpholinreste.

Die hydrierten heterocyclischen Verbindungen für den Rest Y können auch einfach oder mehrfach C-ringsubstituiert sein. Als C-Substituenten kommen dabei z. B. Halogene, Hydroxyl, Cyano, Niederalkyl, Niederalkoxy, Niederalkoxycarbonyl, Acyl mit 1 bis 8 Kohlenstoffatomen, vorzugsweise Niederalkanoyl, Alkylen, Cycloalkyl, Benzyl oder Phenyl in Frage, während N-Substituenten beispielsweise $C_1$-$C_{12}$-Alkyl, $C_3$-$C_{12}$-Alkenyl oder Benzyl sind, die jeweils auch z. B. durch Cyano, Halogen, Hydroxyl, Niederalkyl, Niederalkoxy oder Niederalkoxycarbonyl substituiert sein können. Die Alkyl- und Alkenylreste können geradkettig oder verzweigt sein. Beispiele hierfür sind Methyl, Ethyl, n-Propyl, Isopropyl, n-Butyl, sek.-Butyl, Amyl, n-Hexyl, 2-Ethyl-hexyl, Isooctyl, n-Octyl, Decyl oder n-Dodecyl bzw. Allyl, 2-Methallyl, 2-Ethallyl, 2-Butenyl oder Octenyl.

Der Ring A ist vorzugsweise nicht weiter substituiert. Falls er Substituenten aufweist, ist er in erster Linie durch Halogen, Cyano, Niederalkyl oder Niederalkoxy z. B. durch Cyano, Chlor, Methyl oder Methoxy mono- oder disubstituiert.

Praktisch wichtige chromogene Chinazolinverbindungen entsprechen der Formel

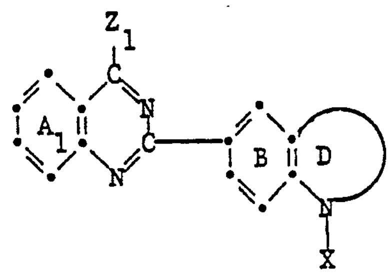

(2)

worin

X Wasserstoff oder unsubstituiertes oder durch Halogen, Cyano oder Niederalkoxy substituiertes Alkyl mit höchstens 8 Kohlenstoffatomen, Cycloalkyl oder Benzyl und

$Z_1$ Wasserstoff, $R_4$, —$OR_4$, —$SR_4$ oder $NR_5R_6$ bedeuten, wobei

$R_4$ unsubstituiertes oder durch Niederalkoxy substituiertes Alkyl mit höchstens 8 Kohlenstoffatomen, Cyclohexyl, Phenyl, Naphthyl, Benzyl oder durch Halogen, Cyano, Niederalkyl, Niederalkoxy oder Niederalkoxycarbonyl substituiertes Phenyl oder Benzyl,

$R_5$ und $R_6$ unabhängig voneinander Wasserstoff, Niederalkyl, Phenyl, Niederalkylphenyl, Niederalkoxyphenyl oder Benzyl und $R_5$ auch Niederalkylcarbonyl, Niederalkylsulfonyl, Benzoyl oder Phenylsulfonyl oder

$R_5$ und $R_6$ zusammen mit dem sie verbindenden Stickstoffatom, Pyrrolidino, Piperidino oder Morpholino bedeuten, und worin die Ringe $A_1$ und B, unabhängig voneinander, unsubstituiert oder durch Cyano, Halogen, Niederalkyl z. B. Methyl oder Niederalkoxy wie Methoxy substituiert sein können und der Ring D einen hydrierten fünf- oder sechsgliedrigen Stickstoffheterocyclus darstellt, der gegebenenfalls als Ringglied ein weiteres Heteroatom, z. B. Sauerstoff, Schwefel oder Stickstoff aufweist und unsubstituiert oder durch Halogen, Cyano, Hydroxyl, Niederalkyl, Niederalkoxy, $C_5$-$C_6$-Cycloalkyl, Benzyl oder $C_3$-$C_6$-Alkylen einfach oder je nach Substituenten mehrfach C-substituiert ist.

Unter den Chinazolinverbindungen der Formel (2) sind diejenigen, in denen $Z_1$ —$OR_4$ bedeutet, bevorzugt. $R_4$ ist vorzugsweise Niederalkyl, Benzyl, oder vor allem Phenyl. X ist vorzugsweise Niederalkyl, Benzyl oder β-Cyanoethyl. Der Ring D ist vorzugsweise sechsgliedrig und vor allem durch 1, 2 oder 3 Methylgruppen C-substituiert. Die Ringe $A_1$ und B sind vorzugsweise unsubstituiert. Der Ring B kann jedoch vorteilhafterweise eine Methylgruppe aufweisen.

Von grossem Interesse sind Chinazolinverbindungen der Formel

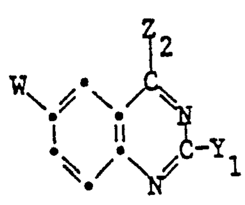

(3)

worin

$Z_2$ Wasserstoff, $R_7$, $—OR_7$, $—SR_7$ oder $—NR_8R_9$,

$R_7$ Niederalkyl, Niederalkoxy-niederalkyl, Cyclohexyl, Phenyl, Naphthyl, Benzyl oder durch Halogen, Cyano, Methyl oder Methoxy substituiertes Phenyl,

$R_8$ und $R_9$ unabhängig voneinander, Wasserstoff, Niederalkyl, Phenyl oder Benzyl und

$R_8$ auch Niederalkylcarbonyl oder Benzoyl,

W Halogen, Methyl, Methoxy oder besonders Wasserstoff und

$Y_1$ einen 5-Indolinylrest der Formel

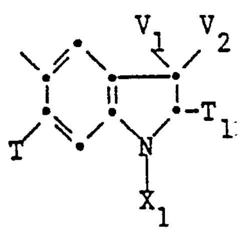

(3a)

einen Tetrahydrochinolinylrest der Formel

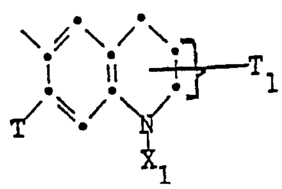

(3b)

einen Tetrahydrochinolinylrest der Formel

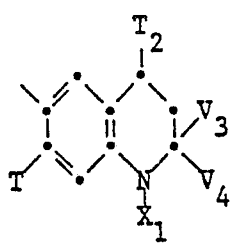

(3c)

oder einen Benzomorpholinorest der Formel

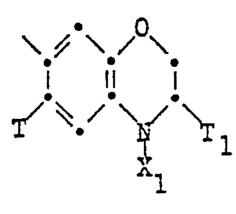

(3d)

$X_1$ Wasserstoff, $C_1$-$C_8$-Alkyl, $C_2$-$C_6$-Alkoxyalkyl, β-Cyanoethyl oder Benzyl,

T Wasserstoff, Halogen, Niederalkyl, Niederalkoxy, $C_1$-$C_4$-Acylamino oder Phenyl,

$T_1$ und $T_2$ je Wasserstoff, Halogen, Hydroxy, Niederalkyl oder Niederalkoxy und

$V_1$, $V_2$, $V_3$ und $V_4$ je Wasserstoff, Niederalkyl, Cycloalkyl oder Benzyl oder ($V_1$ und $V_2$) oder ($V_3$ und $V_4$) je zusammen Alkylen bedeuten.

In Formel (3) sind $R_7$, $R_8$ und $R_9$ vor allem Niederalkyl, Benzyl oder Phenyl. $Z_2$ ist vorzugsweise $—OR_7$.

Der N-Substituent $X_1$ ist insbesondere Benzyl, β-Cyanoethyl oder Alkyl mit 1 bis 8 Kohlenstoffatomen, z. B. n-Octyl, n-Butyl, Isopropyl oder vor allem Methyl oder Ethyl.

$Y_1$ ist in erster Linie der Tetrahydrochinolinrest der Formel (3c).

T ist vorzugsweise Wasserstoff oder Methyl.

$T_1$ ist vorzugsweise Wasserstoff, Methyl, Hydroxyl oder Chlor.

$T_2$ ist vorzugsweise Wasserstoff, Methyl oder Ethyl.

$V_1$ und $V_2$ bedeuten vorzugsweise Wasserstoff oder Methyl.

$V_3$ und $V_4$ bedeuten vorzugsweise jeweils Niederalkyl und insbesondere jeweils Methyl.

Bedeuten ($V_1$ und $V_2$) oder ($V_3$ und $V_4$) zusammen Alkylen, so weisen sie mit Vorteil 4 oder 5 Kohlenstoffatome auf und bilden mit dem sie verbindenden Kohlenstoffatom einen Cyclopentan- oder Cyclohexanring. W ist vorzugsweise Wasserstoff, Halogen oder Niederalkoxy, z. B. Chlor oder Methoxy.

Besonders bevorzugt sind Chinazolinverbindungen der Formel

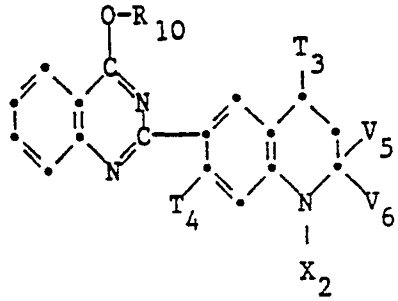

(4)

worin

$R_{10}$ Niederalkyl, Benzyl oder vorzugsweise Phenyl,

$X_2$ Alkyl mit 1 bis 8 Kohlenstoffatomen, β-Cyanoethyl oder Benzyl,

$T_3$, $V_5$ und $V_6$ je Niederalkyl, insbesondere Methyl oder Ethyl und

$T_4$ Wasserstoff oder Methyl bedeuten.

Die erfindungsgemässen Chinazolinverbindungen der Formel (1), in der Z $—OR_1$, $—SR_1$ oder $—NR_2R_3$ bedeutet, werden dadurch hergestellt, dass man eine 4-Halogenchinazolinverbindung der Formel

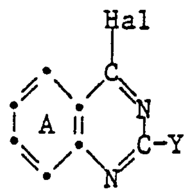

(5)

worin A und Y die angegebene Bedeutung haben und Hal Halogen, wie z. B. Brom, Fluor oder vorzugsweise Chlor bedeutet, mit einer Verbindung der Formel

$R_1-OH$, (6a) $R_1-SH$ (6b) oder 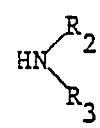 (6c)

umsetzt, worin $R_1$, $R_2$ und $R_3$ die angegebene Bedeutung haben.

Die Umsetzung der Verbindung der Formel (5) mit der Verbindung der Formel (6a), (6b) oder (6c) erfolgt zweckmässig in Anwesenheit eines säurebindenden Mittels, wie z. B. eines Alkalimetallhydroxides, Alkalimetallcarbonates oder einer tertiären Stickstoffbase, wie z. B. Pyridin oder Trialkylaminen, und vorzugsweise in Gegenwart auch eines quaternären Ammoniumsalzes, wie z. B. Tetrabutylammoniumbromides, gegebenenfalls in einem organischen Lösungsmittel oder in einem wässerig-organischen zweiphasigen Medium und bei Rückflusstemperatur.

Als Lösungsmittel kommen beispielsweise cycloaliphatische oder aromatische Kohlenwasserstoffe, wie z. B. Cyclohexan, Benzol, Toluol oder Xylol ; Chlorkohlenwasserstoffe, wie Chloroform, Ethylenchlorid oder Chlorbenzole insbesondere Dichlorbenzol ; Ether, wie Diethylether oder Glykoldimethylether ; cyclische Ether, wie z. B. Dioxan oder Tetrahydrofuran ; sowie Dimethylformamid, Diethylformamid, Dimethylsulfoxid oder Acetonitril in Betracht.

Eine Ausführungsform zur Herstellung von Verbindungen der Formel (1), in der Z Wasserstoff bedeutet, besteht darin, dass man die 4-Halogen-chinazolinverbindung der Formel (5) alkalisch enthalogeniert, wobei das Halogenatom durch Wasserstoff ersetzt wird. Diese Enthalogenierung erfolgt beispielsweise gemäss J. Chem. Soc. 1962, 561-572 unter Verwendung von Toluol-p-sulphonylhydrazid und Zersetzung des entstehenden 4-(N-Toluol-p-sulphonylhydrazino-) chinazolins mit Alkalien, z. B. Natriumhydroxyd, vorzugsweise in Ethylenglykol oder Ethylenglykolmonomethylether.

Die Ausgangsstoffe der Formel (5) können dadurch hergestellt werden, dass man beispielsweise ein 2-Amino-benzoesäureamid der Formel

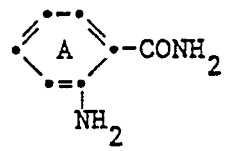
(7)

mit einem Aldehyd der Formel

Y—CHO (8)

zu einer 1,2,3,4-Tetrahydro-Chinazolon(4)-Verbindung der Formel

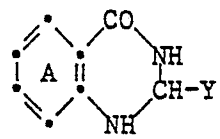
(9)

umsetzt, diese zu einer Verbindung der Formel

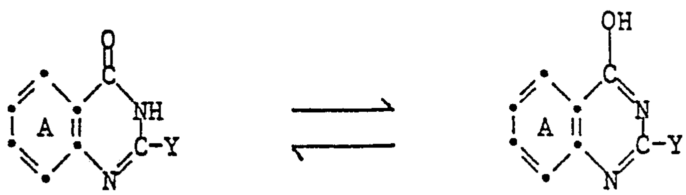
(10)

oxidiert, sodann die Hydroxylgruppe am heterocyclischen Ring des Chinazolinsystems durch ein Halogenatom, z. B. mittels Phosphoroxichlorid in Dichlorbenzol oder Thionylchlorid in Dimethylformamid unter Bildung des Ausgangsstoffes der Formel (5) ersetzt. Die erhaltene 4-Halogenchinazolinverbindung kann, ohne isoliert zu werden, weiterverwendet werden.

Die Oxidation der Umsetzungsprodukte der Formel (9) zu den 4-Chinazolon-verbindungen der Formel (10) erfolgt mit Oxidationsmitteln. Geeignete Oxidationsmittel sind z. B. Chromate, Bichromate, Chlorate, Chlorite, Peroxide, z. B. Wasserstoffperoxid, Mangandioxid, Bleidioxid, molekularer Sauerstoff, Luft, Perborate, Permanganate, Nitrite, Chlor, Brom und vor allem Chloranil oder Bisulfite.

Die besten Ergebnisse in Bezug auf Ausbeute und Reinheit der erhaltenen 4-Chinazolon-verbindungen erzielt man mit Chloranil als bevorzugtes Oxidationsmittel. Oekologische Vorteile bietet die Oxidation mit Natriumbisulfit. Auf analoge Weise wie in Synthesis 1981, (1), 35 beschrieben, erhält man unter Verwendung dieses Oxidationsmittels Chinazolonverbindungen der Formel (10) in guter Reinheit und Ausbeute.

Ein bevorzugtes Verfahren zur Herstellung von Verbindungen der Formel (1), in der Z R bedeutet, besteht darin, dass man eine Ketoamidoverbindung der Formel

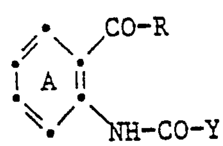
(11)

worin A, R und Y die angegebene Bedeutung haben, mit einer alkoholischen, vorzugsweise methanolischen Ammoniaklösung zu einer Chinazolinverbindung der Formel

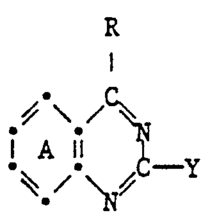
(12)

reagieren lässt. Die Umsetzung der Ketoamidoverbindung der Formel (11) mit der Ammoniaklösung kann bei einer Temperatur von 80 bis 200 °C, vorzugsweise 100 bis 180 °C, vorgenommen werden. Verbindungen der Formel (11) können gemäss A. Bischler und D. Barad, Ber., 25, 3080 (1982) und A. Bischler und F.J. Howell, Ber. 26, 1384 (1893) durch Acylierung der entsprechenden Ketoaminoverbindungen mit den gewünschten Säureanhydriden oder Säurehalogeniden hergestellt werden.

Die Chinazolinverbindungen der Formel (1) bis (4) sind normalerweise farblos oder schwach gefärbt. Wenn diese Farbbildner mit einem vorzugsweise sauren Entwickler, z. B. einem Elektronenakzeptor, in Kontakt gebracht werden, so ergeben sie je nach Bedeutung von Y und Z intensive, gelbe, orange oder rote Farbtöne, die ausgezeichnet sublimations- und lichtecht sind. Sie sind deshalb auch sehr wertvoll im Gemisch mit einem oder mehreren anderen bekannten Farbbildnern, z. B. 3,3-(Bisaminophenyl-)phthaliden, 3-Indolyl-3-aminophenyl-azaphthaliden, 3,3-(Bis-indolyl)-phthaliden, 3-Aminofluoranen, 2,6-Diaminofluoranen, Leukoauraminen, Spiropyranen, Dispiropyranen, Phenoxazinen, Phenothiazinen, Carbazolylmethanen, weiteren Triarylmethan-leukofarbstoffen, um blaue, marine-blaue, graue oder schwarze Färbungen zu ergeben.

Die Chinazolinverbindungen der Formeln (1) bis (4) zeigen sowohl auf phenolischen Unterlagen, wie auch besonders auf Tonen, eine ausgezeichnete Farbintensität und Lichtechtheit. Sie eignen sich vor allem als sich schnell entwickelnde Farbbildner für die Verwendung in einem wärmeempfindlichen oder insbesondere druckempfindlichen Aufzeichnungsmaterial, das sowohl Kopier- als auch Registriermaterial sein kann. Sie zeichnen sich dadurch aus, dass sie in den Kapselölen hervorragend löslich sind und durch Belichtung im CB-Blatt eine geringe Abnahme der Farbstärke (CB-Desaktivierung) aufweisen.

Ein druckempfindliches Material besteht beispielsweise aus mindestens einem Paar von Blättern, die mindestens einen Farbbildner der Formeln (1) bis (4) gelöst in einem organischen Lösungsmittel und einen Elektronenakzeptor als Entwickler enthalten.

Typische Beispiele für solche Entwickler sind Aktivton-Substanzen, wie Attapulgus-Ton, Säureton, Bentonit, Montmorillonit, aktivierter Ton, wie z. B. säureaktiviertes Bentonit oder Montmorillonit, ferner Zeolith, Halloysit, Siliciumdioxyd, Aluminiumoxid, Aluminiumsulfat, Aluminiumphosphat, Zinkchlorid, Zinknitrat, Kaolin oder irgendein beliebiger Ton oder sauer reagierende, organische Verbindung, wie z. B. gegebenenfalls ringsubstituierte Phenole, Salicylsäure oder Salicylsäureester und deren Metallsalze, ferner ein sauer reagierendes, polymeres Material, wie z. B. ein phenolisches Polymerisat, ein Alkylphenolacetylenharz, ein Maleinsäure-Kolophonium-Harz oder ein teilweise oder vollständig hydrolysiertes Polymerisat von Maleinsäureanhydrid mit Styrol, Ethylen oder Vinylmethylether, oder Carboxypolymethylen. Es können auch Mischungen der genannten polymeren Verbindungen eingesetzt werden. Bevorzugte Entwickler sind säureaktiviertes Bentonit, Zinksalicylate oder die Kondensationsprodukte von p-substituierten Phenolen mit Formaldehyd. Die letzteren können auch Zink enthalten.

Die Entwickler können zusätzlich auch mit an sich unreaktiven oder wenig reaktiven Pigmenten oder weiteren Hilfsstoffen wie Kieselgel oder UV-Adsorbern, wie z. B. 2-(2-Hydroxiphenyl-)benzotriazolen gemischt eingesetzt werden. Beispiele für solche Pigmente sind :

Talk, Titandioxid, Zinkoxid, Kreide ; Tone wie Kaolin, sowie organische Pigmente, z. B. Harnstoff-Formaldehyd-Kondensate (BET-Oberfläche 2-75 $m^2/g$) oder Melamin-Formaldehyd-Kondensationsprodukte.

Der Farbbildner liefert an den Punkten, an denen er mit dem Elektronenakzeptor in Kontakt kommt, eine gefärbte Markierung. Um eine frühzeitige Aktivierung der in dem druckempfindlichen Aufzeichnungsmaterial vorhandenen Farbbildner zu verhindern, werden diese in der Regel von dem Elektronenakzeptor getrennt. Dies kann zweckmässig erzielt werden, indem man die Farbbildner in schaum-, schwamm- oder bienenwabenartigen Strukturen einarbeitet. Vorzugsweise sind die Farbbildner in Mikrokapseln eingeschlossen, die sich in der Regel durch Druck zerbrechen lassen.

Beim Zerbrechen der Kapseln durch Druck, beispielsweise mittels eines Bleistiftes wird die Farbbildnerlösung auf ein benachbartes, mit einem Elektronenakzeptor beschichtetes Blatt übertragen, wodurch eine farbige Stelle erzeugt wird. Die Farbe resultiert aus dem dabei gebildeten Farbstoff, der im sichtbaren Bereich des elektromagnetischen Spektrums absorbiert.

Die Farbbildner werden vorzugsweise in Form von Lösungen in organischen Lösungsmitteln eingekapselt. Beispiele für geeignete Lösungsmittel sind vorzugsweise nichtflüchtige Lösungsmittel, z. B. polyhalogeniertes Paraffin oder Diphenyl, wie Chlorparaffin, Monochlordiphenyl oder Trichlordiphenyl, ferner Tricresylphosphat, Di-n-butylphthalat, Dioctylphthalat, Trichlorbenzol, Trichlorethylphosphat, aromatische Ether, wie Benzylphenylether, Kohlenwasserstofföle, wie Paraffin oder Kerosin, z. B. mit Isopropyl, Isobutyl, sek.Butyl oder tert.Butyl alkylierte Derivate von Diphenyl, Naphthalin oder Terphenyl, Dibenzyltoluol, Terphenyl, partiell hydriertes Terphenyl, mono- bis tetramethylierte Diphenylalkane, benzylierte Xylole, oder weitere chlorierte oder hydrierte, kondensierte, aromatische Kohlenwasserstoffe. Oft werden Mischungen verschiedener Lösungsmittel, insbesondere Mischungen aus Paraffinölen oder Kerosin und Diisopropylnaphthalin oder partiell hydriertem Terphenyl, eingesetzt, um eine optimale Löslichkeit für die Farbbildner, eine rasche und intensive Färbung und eine für die Mikroverkapselung günstige Viskosität zu erreichen.

Die Kapselwände können durch Koazervationskräfte gleichmässig um die Tröpfchen der Farbbildnerlösung herum gebildet werden, wobei das Einkapselungsmaterial z. B. aus Gelatine und Gummi arabicum bestehen kann, wie dies z. B. in der US-Patentschrift 2 800 457 beschrieben ist. Die Kapseln

können vorzugsweise auch aus einem Aminoplast oder modifizierten Aminoplasten durch Polykondensation gebildet werden, wie es in den britischen Patentschriften 989, 264, 1 156 725, 1 301 052 und 1 355 124 beschrieben ist. Ebenfalls geeignet sind Mikrokapseln, welche durch Grenzflächenpolymerisation gebildet werden, wie z. B. Kapseln aus Polyester, Polycarbonat, Polysulfonamid, Polysulfonat, besonders aber aus Polyamid oder Polyurethan.

Die Farbbildner der Formel (1) enthaltenden Mikrokapseln können zur Herstellung von druckempfindlichen Kopiermaterialien der verschiedensten bekannten Arten verwendet werden. Die verschiedenen Systeme unterscheiden sich im wesentlichen voneinander durch die Anordnung der Kapseln, der Farbreaktanten und durch das Trägermaterial. Bevorzugt wird eine Anordnung, bei der der eingekapselte Farbbildner in Form einer Schicht auf der Rückseite eines Uebertragungsblattes und der Elektronenakzeptor in Form einer Schicht auf der Vorderseite eines Empfangsblattes vorhanden sind.

Eine andere Anordnung der Bestandteile besteht darin, dass die den Farbbildner enthaltenden Mikrokapseln und der Entwickler in oder auf dem gleichen Blatt in Form einer oder mehrerer Einzelschichten oder in der Papierpulpe vorliegen.

Die Kapseln werden vorzugsweise mittels eines geeigneten Binders auf dem Träger befestigt. Da Papier das bevorzugte Trägermaterial ist, handelt es sich bei diesem Binder hauptsächlich um Papierbeschichtungsmittel, wie Gummi arabicum, Polyvinylalkohol, Hydroxymethylcellulose, Casein, Methylcellulose, Dextrin, Stärke oder Stärkederivate oder Polymerlatices. Letztere sind beispielsweise Butadien-Styrolcopolymerisate oder Acrylhomo- oder -copolymere.

Als Papier werden nicht nur normale Papiere aus Cellulosefasern, sondern auch Papiere, in denen die Cellulosefasern (teilweise oder vollständig) durch Fasern aus synthetischen Polymerisaten ersetzt sind, verwendet.

Die Verbindungen der Formeln (1) bis (4) können auch als Farbbildner in einem thermoreaktiven Aufzeichnungsmaterial verwendet werden. Dieses enthält in der Regel mindestens einen Schichtträger, einen Farbbildner, einen Elektronenakzeptor und gegebenenfalls auch ein Bindemittel und/oder Wachs.

Thermoreaktive Aufzeichnungssysteme umfassen z. B. wärmeempfindliche Aufzeichnungs- und Kopiermaterialien und -papiere. Diese Systeme werden beispielsweise zum Aufzeichnen von Informationen, z. B. in elektronischen Rechnern, Ferndruckern, Fernschreibern oder in Aufzeichnungsgeräten und Messinstrumenten, wie z. B. Elektrocardiographen, verwendet. Die Bilderzeugung (Markierung) kann auch manuell mit einer erhitzten Feder erfolgen. Eine weitere Einrichtung der Erzeugung von Markierungen mittels Wärme sind Laserstrahlen.

Das thermoreaktive Aufzeichnungsmaterial kann so aufgebaut sein, dass der Farbbildner in einer Bindemittelschicht gelöst oder dispergiert ist und in einer zweiten Schicht der Entwickler in dem Bindemittel gelöst und dispergiert ist. Eine andere Möglichkeit besteht darin, dass sowohl der Farbbildner als auch der Entwickler in einer Schicht dispergiert sind. Das Bindemittel wird in spezifischen Bezirken mittels Wärme erweicht und an diesen Punkten, an denen Wärme angewendet wird, kommt der Farbbildner mit dem Elektronenakzeptor in Kontakt und es entwickelt sich sofort die erwünschte Farbe.

Als Entwickler eignen sich die gleichen Elektronenakzeptoren, wie sie in druckempfindlichen Papieren verwendet werden. Beispiele für Entwickler sind die bereits erwähnten Tonminerale und Phenolharze, oder auch phenolische Verbindungen, wie sie beispielsweise in der DE-PS 12 51 348 beschrieben sind, z. B. 4-tert.-Butylphenol, 4-Phenylphenol, Methylen-bis-(p-phenylphenol), 4-Hydroxydiphenylether, α-Naphthol, β-Naphthol, 4-Hydroxybenzoesäuremethylester, 4-Hydroxyacetophenon, 2,2'-Dihydroxydiphenyl, 4,4'-Isopropylidendiphenol, 4,4'-Isopropyliden-bis-(2-methylphenol), 4,4'-Bis-(hydroxyphenyl)-valeriansäure, Hydrochinon, Pyrogallol, Phloroglucin, p-, m-, o-Hydroxybenzoesäure, Gallussäure, 1-Hydroxy-2-naphthoesäure sowie Borsäure und organische, vorzugsweise aliphatische Dicarbonsäuren, wie z. B. Weinsäure, Oxalsäure, Maleinsäure, Zitronensäure, Citraconsäure oder Bernsteinsäure.

Vorzugsweise werden zur Herstellung des thermoreaktiven Aufzeichnungsmaterials schmelzbare, filmbildende Bindemittel verwendet. Diese Bindemittel sind normalerweise wasserlöslich, während die Chinazolinverbindungen und der Entwickler in Wasser schwer löslich oder unlöslich sind. Das Bindemittel sollte in der Lage sein, den Farbbildner und den Entwickler bei Raumtemperatur zu dispergieren und zu fixieren.

Bei Einwirkung von Wärme erweicht oder schmilzt das Bindmittel, so dass der Farbbildner mit dem Entwickler in Kontakt kommt und sich eine Farbe bilden kann. Wasserlösliche oder mindestens in Wasser quellbare Bindemittel sind z. B. hydrophile Polymerisate, wie Polyvinylalkohol, Polyacrylsäure, Hydroxyethylcellulose, Methylcellulose, Carboxymethylcellulose, Polyacrylamid, Polyvinylpyrrolidon, Gelatine, Stärke oder veretherte Maisstärke.

Wenn der Farbbildner und der Entwickler in zwei getrennten Schichten vorliegen, können in Wasser unlösliche Bindemittel, d. h. in nichtpolaren oder nur schwach polaren Lösungsmitteln lösliche Bindemittel, wie z. B. Naturkautschuk, synthetischer Kautschuk, chlorierter Kautschuk, Alkydharze, Polystyrol, Styrol/Butadien-Mischpolymerisate, Polymethylacrylate, Ethylcellulose, Nitrocellulose und Polyvinylcarbazol, verwendet werden. Die bevorzugte Anordnung ist jedoch diejenige, bei der der Farbbildner und der Entwickler in einer Schicht in einem wasserlöslichen Bindemittel enthalten sind.

Die thermoreaktiven Schichten können weitere Zusätze enthalten. Zur Verbesserung des Weissgrades, zur Erleichterung des Bedruckens der Papiere und zur Verhinderung des Festklebens der erhitzten Feder können diese Schichten, z. B. Talk, Titandioxyd, Zinkoxyd, Aluminiumhydroxyd, Calciumcarbonat,

Tone oder auch organische Pigmente, wie z. B. Harnstoff-Formaldehydpolymerisate, enthalten. Um zu bewirken, dass nur innerhalb eines begrenzten Temperaturbereiches die Farbe gebildet wird, können Substanzen, wie Harnstoff, Thioharnstoff, Diphenylthioharnstoff, Acetamid, Acetanilid, Stearinsäureamid, Phthalsäureanhydrid, Metallstearate, Phthalsäurenitril oder andere entsprechende, schmelzbare Produkte, welche das gleichzeitige Schmelzen des Farbbildners und des Entwicklers induzieren, zugesetzt werden. Bevorzugt enthalten thermographische Aufzeichnungsmaterialien Wachse, z. B. Carnaubawachs, Montanawachs, Paraffinwachs, Polyethylenwachs, Kondensate höherer Fettsäureamide und Formaldehyd und Kondensate höherer Fettsäuren und Ethylendiamin.

In den folgenden Beispielen beziehen sich die angegebenen Prozentsätze, wenn nichts anderes angegeben ist, auf das Gewicht.

Beispiel 1

19 g der Chinazolonverbindung der Formel

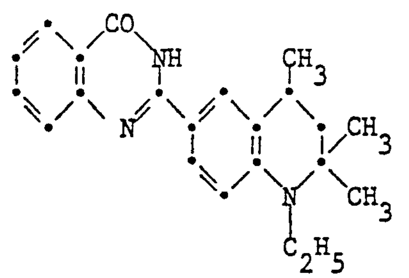

(i)

werden bei 150 °C in 90 g 1,2-Dichlorbenzol gelöst. Hierauf lässt man die Lösung auf 95 °C abkühlen und 9 g Phosphoroxitrichlorid im Verlauf von 30 Minuten bei dieser Temperatur zutropfen. Alsdann wird die Reaktionsmischung noch 1 Stunde bei 90-95 °C gerührt. Die entstandene rote Lösung der 4-Chlor-2-tetrahydrochinolinyl-chinazolin-verbindung der Formel

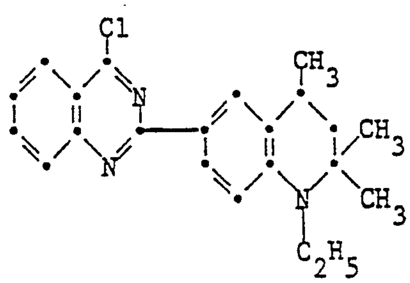

(ii)

wird im Verlauf von 15 Minuten auf eine Suspension bestehend aus 5,2 g Phenol, 2,2 g Tetrabutylammoniumbromid und 35 g einer 50 %igen wässerigen Natriumhydroxidlösung gegossen, worauf die Temperatur auf 110 °C ansteigt. Man rührt die Reaktionsmischung noch 1 Stunde bei 100-110 °C und entfernt das 1,2-Dichlorbenzol durch Wasserdampfdestillation. Das ausgefallene Produkt wird unter Erwärmen in Toluol gelöst und die heisse Toluollösung über Aktivkohle filtriert. Beim Abkühlen kristallisiert das Produkt aus und wird abfiltriert und getrocknet. Man erhält 20 g einer Verbindung der Formel

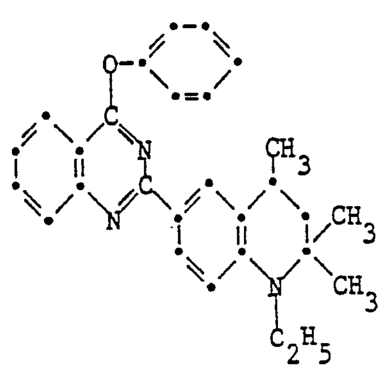

(21)

mit einem Schmelzpunkt von 145-147 °C.

Auf Säureton entwickelt dieser Farbbildner eine rotstichig gelbe Farbe mit guter Licht- und Sublimierechtheit. Remissionsmaximum 477 nm.

Die im Beispiel 1 verwendete Chinazolonverbindung der Formel (i) kann wie folgt hergestellt werden :

23,1 g N-Ethyl-2,2,4-trimethyl-tetrahydrochinolin-6-aldehyd werden in 150 ml Ethanol gelöst. Man gibt zur Lösung 13,6 g Anthranilsäureamid und 4 ml Schwefelsäure (10 %) zu und erwärmt die Reaktionsmischung auf 60 °C. Man hält die Mischung 1 Stunde bei 60 °C und oxidiert das Produkt durch Zutropfen von 69 g einer 40 %igen wässerigen Natriumbisulfitlösung und anschliessendes Rühren während 2 Stunden bei Rückflusstemperatur. Nach Abkühlung auf Raumtemperatur wird das ausgefallene Produkt abfiltriert, mit Ethanol gewaschen und getrocknet. Man erhält 19 g der Chinazolonverbindung der Formel (i) mit einem Schmelzpunkt von 215-219 °C.

Auf gleiche Weise wie in Beispiel 1 beschrieben, erhält unter Verwendung der entsprechenden Ausgangsstoffe die in der folgenden Tabelle aufgeführten Farbbildner der Formel

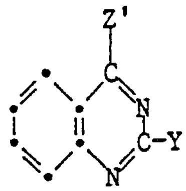

(22)

Tabelle

| Beispiel Nr. | Z' | Y' | Remissionsmaximum auf Säureton in nm | Farbe |
|---|---|---|---|---|
| 2 | $-OCH_3$ | 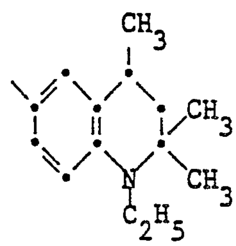 | 467 | gelb |
| 3 | 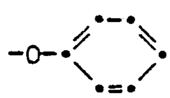 | 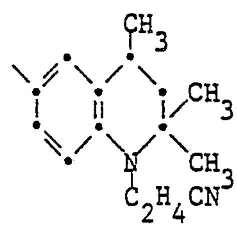 Smp. 175-181 °C | 451 | gelb |
| 4 | 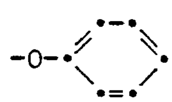 | 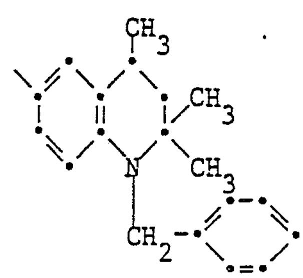 | 471 | gelb |
| 5 | 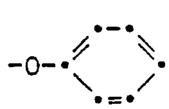 | 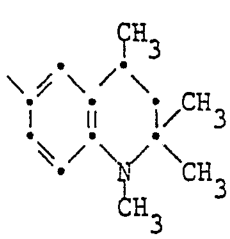 Smp. 113-128 °C | 476 | gold-gelb |

Tabelle (Fortsetzung)

| Beispiel Nr. | Z' | Y' | Remissions-maximum auf Säureton in nm | Farbe |
|---|---|---|---|---|
| 6 | 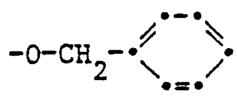 | 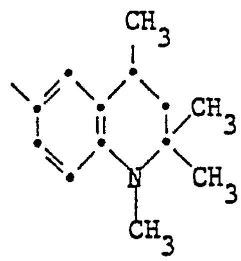 Smp. 164-166°C | 468 | gelb |
| 7 | 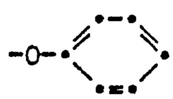 | 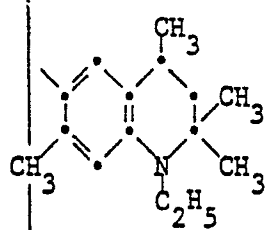 Smp. 139-140°C | 482 | goldgelb |
| 8 | 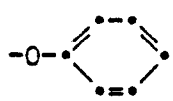 | 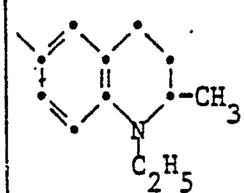 Smp. 103-106°C | 480 | gold-gelb |
| 9 | 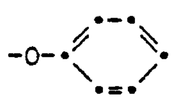 | 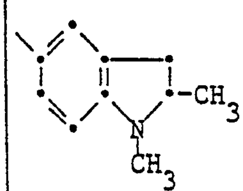 | 483 | orange |
| 10 | 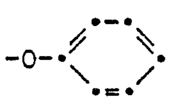 | 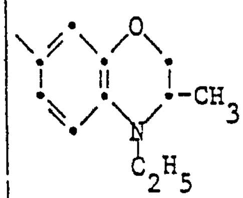 | 480 | goldgelb |

Beispiel 11

Herstellung eines druckempfindlichen Kopierpapiers

Eine Lösung von 3 g der Chinazolinverbindung der Formel (21) in 80 g partiell hydriertem Terphenyl und 17 g Kerosin wird auf an sich bekannte Weise mit Gelatine und Gummi arabicum durch Koazervation mikroverkapselt, mit Stärkelösung vermischt und auf ein Blatt Papier gestrichen. Ein zweites Blatt Papier wird auf der Frontseite mit säureaktiviertem Bentonit als Farbentwickler beschichtet. Das erste, den Farbbildner enthaltende Blatt und das mit Farbentwickler beschichtete Papier werden mit den Beschichtungen benachbart aufeinandergelegt. Durch Schreiben mit der Hand oder mit der Schreibmaschine auf dem ersten Blatt wird Druck ausgeübt, und es entwickelt sich sofort auf dem mit dem Entwickler beschichteten Blatt eine intensive rotstichig gelbe Kopie, die ausgezeichnet sublimier- und lichtecht ist.

Entsprechende intensive, sublimier- und lichtechte gelbe oder orange Kopien werden auch bei Verwendung jedes der anderen in den Herstellungsbeispielen angegebenen Farbbildner der Beispiele 2 bis 10 erzielt.

Beispiel 12

Ersetzt man in Beispiel 11 die Chinazolinverbindung der Formel (21) durch eine Mischung der folgenden Zusammensetzung

1,2 g 3,3-Bis-(4'-dimethylaminophenyl)-6-dimethylaminophthalid,
1,2 g N-Butylcarbazol-3-yl-bis-(4'-N-methyl-N-phenylaminophenyl-)methan
1,2 g der Chinazolinverbindung der Formel (21) und
0,4 g 3,3-Bis-(N-n-octyl-2'-methylindol-3'-yl-)phthalid

und verfährt im übrigen wie in Beispiel 11 beschrieben, so erhält man ein druckempfindliches Aufzeichnungsmaterial, welches durch Schreiben mit der Hand oder mit der Schreibmaschine eine intensive und lichtechte schwarze Kopie ergibt.

Beispiel 13

1 g der Chinazolinverbindung der Formel (21) wird in 17 g Toluol gelöst. Zu dieser Lösung gibt man unter Rühren 12 g Polyvinylacetat, 8 g Calciumcarbonat und 2 g Titandioxyd. Die erhaltene Suspension wird im Gewichtsverhältnis 1 : 1 mit Toluol verdünnt und mit einem 10 µm Rakel auf ein Blatt Papier gestrichen. Auf dieses Blatt Papier wird ein zweites Blatt Papier gelegt, dessen Unterseite bei einem Auftragsgewicht von 3 $g/m^2$ mit einer Mischung bestehend aus 1 Teil eines Amidwachses, 1 Teil eines Stearinwachses und 1 Teil Zinkchlorid beschichtet worden ist. Durch Schreiben mit der Hand oder mit der Schreibmaschine auf dem oberen Blatt wird Druck ausgeübt, und es entwickelt sich sofort auf dem mit den Farbbildner beschichteten Blatt, eine intensive, sublimier- und lichtechte rotstichig gelbe Farbe.

Beispiel 14

Herstellung eines wärmeempfindlichen Aufzeichnungsmaterials

In einer Kugelmühle werden 32 g 4,4'-Isopropylidendiphenol (Bisphenol A), 3,8 g Distearylamid des Ethylendiamins, 39 g Kaolin, 20 g eines zu 88 % hydrolysierten Polyvinylalkohols und 500 ml Wasser gemahlen bis die Teilchengrösse ca. 5 µm beträgt. In einer zweiten Kugelmühle werden 6 g der Verbindung der Formel (21), 3 g eines zu 88 % hydrolysierten Polyvinylalkohols und 60 ml Wasser zu einer Teilchengrösse von ca. 3 µm gemahlen.

Die beiden Dispersionen werden zusammengegeben und mit einem Trockenauftragsgewicht von 5,5 $g/m^2$ auf ein Papier gestrichen. Durch Berührung des Papiers mit einem erhitzten Kugelschreiber wird eine intensive gelbe Farbe erhalten, die eine ausgezeichnete Sublimier- und Lichtechtheit hat.

Intensive und lichtechte gelbe Farben können auch bei Verwendung jedes der anderen Farbbildner der Beispiele 2 bis 10 erhalten werden.

Beispiel 15

In einer Kugelmühle werden 2,7 g der Chinazolinverbindung der Formel (21), 24 g N-Phenyl-N'-(1-hydroxy-2,2,2-trichlorethyl)-harnstoff, 16 g Stearinsäureamid, 59 g eines zu 88 % hydrolysierten Polyvinylalkohols und 58 ml Wasser gemahlen bis die Teilchengrösse 2-5 µm beträgt. Diese Suspension wird bei einem Trockenauftragsgewicht vom 5,5 $g/m^2$ auf ein Blatt Papier gestrichen. Durch Berührung des Papiers mit einem erhitzten Kugelschreiber wird eine intensive, sublimier- und lichtechte gelbe Farbe erhalten.

**Patentansprüche**

1. Chromogene Chinazolinverbindungen der Formel

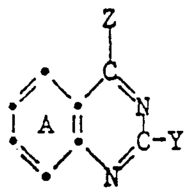

(1)

worin

Y den Rest eines hydrierten stickstoffhaltigen heterocyclischen Verbindung, die über einen in ihr enthaltenden ankondensierten Benzolkern mit dem Chinazolinbestandteil gebunden und unsubstituiert oder substituiert ist und

Z Wasserstoff, R, $—OR_1$, $—SR_1$ oder $—NR_2R_3$ bedeuten, wobei

R und $R_1$ je unsubstituiertes oder durch Cyano oder Niederalkoxy substituiertes Alkyl mit höchstens 12 Kohlenstoffatomen, Cycloalkyl, Phenyl, Naphthyl, Diphenyl, Benzyl oder Phenylethyl, die jeweils unsubstituiert oder durch Halogen, Cyano, Nitro, Niederalkyl, Niederalkoxy, Niederalkoxycarbonyl oder Niederalkylcarbonyl substituiert sind, einen fünf- oder sechsgliedrigen, heterocyclischen Rest aromatischen Charakters, der unsubstituiert oder durch Halogen, Cyano, Niederalkyl, Niederalkoxy oder Niederalkoxycarbonyl substituiert ist und $R_1$ auch Halogenalkyl mit 2 bis 6 Kohlenstoffatomen,

$R_2$ und $R_3$ unabhängig voneinander, je Wasserstoff, unsubstituiertes oder durch Halogen, Hydroxy, Cyano oder Niederalkoxy substituiertes Alkyl mit höchstens 12 Kohlenstoffatomen, oder Cycloalkyl, Phenyl, Benzyl oder durch Halogen, Nitro, Cyano, Niederalkyl, Niederalkoxy oder Niederalkoxycarbonyl substituiertes Phenyl oder Benzyl und $R_2$ auch Acyl mit 1 bis 12 Kohlenstoffatomen, oder

$R_2$ und $R_3$ zusammen mit dem sie verbindenden Stickstoffatom einen fünf- oder sechsgliedrigen, gesättigten heterocyclischen Rest bedeuten, und der Ring

A unsubstituiert oder durch Halogen, Cyano, Nitro, Niederalkyl, Niederalkoxy oder Niederalkoxycarbonyl substituiert ist, wobei der Ausdruck «Nieder» sich auf solche Gruppen mit 1 bis 5 Kohlenstoffatomen bezieht.

2. Chinazolinverbindungen gemäss Anspruch 1, dadurch gekennzeichnet, dass in Formel (1) Z den Rest $—OR_1$ darstellt.

3. Chinazolinverbindungen gemäss einem der Ansprüche 1 und 2, dadurch gekennzeichnet, dass in Formel (1) Y einen Indolin-, Tetrahydrochinolin- oder Benzomorpholinrest darstellt.

4. Chinazolinverbindungen gemäss Anspruch 1, dadurch gekennzeichnet, dass sie der Formel

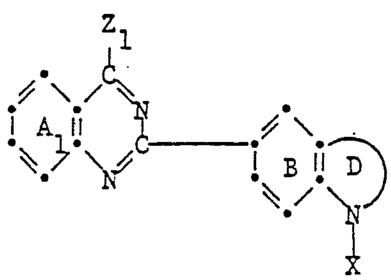

(2)

entsprechen, worin

X Wasserstoff oder unsubstituiertes oder durch Halogen, Cyano oder Niederalkoxy substituiertes Alkyl mit höchstens 8 Kohlenstoffatomen, Cycloalkyl oder Benzyl und

$Z_1$ Wasserstoff, $R_4$, $—OR_4$, $—SR_4$ oder $NR_5R_6$ bedeuten, wobei

$R_4$ unsubstituiertes oder durch Niederalkoxy substituiertes Alkyl mit höchstens 8 Kohlenstoffatomen, Cyclohexyl, Phenyl, Naphthyl, Benzyl oder durch Halogen, Cyano, Niederalkyl, Niederalkoxy oder Niederalkoxycarbonyl substituiertes Phenyl oder Benzyl,

$R_5$ und $R_6$, unabhängig voneinander, je Wasserstoff, Niederalkyl, Phenyl, Niederalkylphenyl, Niederalkoxyphenyl oder Benzyl und $R_5$ auch Niederalkylcarbonyl, Niederalkylsulfonyl, Benzoyl oder Phenylsulfonyl oder

$R_5$ und $R_6$ zusammen mit dem sie verbindenden Stickstoffatom, Pyrrolidino, Piperidino oder Morpholino bedeuten, und worin die Ringe

$A_1$ und B, unabhängig voneinander, je unsubstituiert oder durch Cyano, Halogen, Niederalkyl oder Niederalkoxy substituiert sind und der Ring

D einen hydrierten fünf- oder sechsgliedrigen Stickstoffheterocyclus darstellt, der gegebenenfalls als Ringglied ein weiteres Heteroatom aufweist und unsubstituiert oder durch Halogen, Cyano, Hydroxyl, Niederalkyl, Niederalkoxy, $C_5$-$C_6$-Cycloalkyl, Benzyl oder $C_3$-$C_6$-Alkylen einfach oder mehrfach C-substituiert ist.

5. Chinazolinverbindungen gemäss Anspruch 4, dadurch gekennzeichnet, dass in Formel (2) $Z_1$ den Rest $—OR_4$ darstellt und $R_4$ Niederalkyl, Benzyl oder Phenyl bedeutet.

6. Chinazolinverbindungen gemäss einem der Ansprüche 4 und 5, dadurch gekennzeichnet, dass in Formel (2) der Ring D sechsgliedrig ist.

7. Chinazolinverbindungen gemäss einem der Ansprüche 4 bis 6, dadurch gekennzeichnet, dass in Formel (2) X Niederalkyl, Benzyl oder β-Cyanoethyl bedeutet.

8. Chinazolinverbindungen gemäss Anspruch 1, dadurch gekennzeichnet, dass sie der Formel

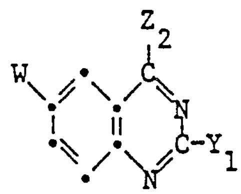

(3)

entsprechen, worin

$Z_2$ Wasserstoff, $R_7$, $—OR_7$, $SR_7$ oder $NR_8R_9$,

$R_7$ Niederalkyl, Niederalkoxy-niederalkyl, Cyclohexyl, Phenyl, Naphthyl, Benzyl oder durch Halogen, Cyano, Methyl oder Methoxy substituiertes Phenyl,

$R_8$ und $R_9$ unabhängig voneinander, Wasserstoff, Niederalkyl, Phenyl oder Benzyl und

$R_8$ auch Niederalkylcarbonyl oder Benzoyl,

W Wasserstoff, Halogen, Methyl oder Methoxy und

$Y_1$ einen 5-Indolinylrest der Formel

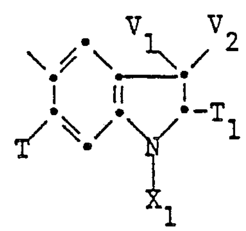

(3a)

einen Tetrahydrochinolinylrest der Formel

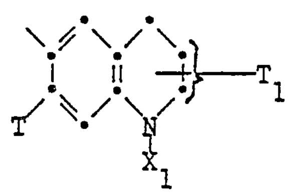

(3b)

einen Tetrahydrochinolinylrest der Formel

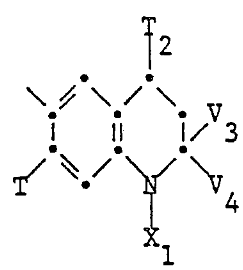

(3c)

oder einen Benzomorpholinorest der Formel

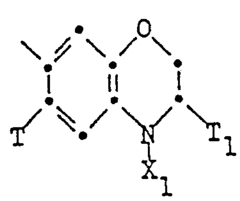

(3d)

$X_1$ Wasserstoff, $C_1$-$C_8$-Alkyl, $C_2$-$C_6$-Alkoxyalkyl, β-Cyanoethyl oder Benzyl,
T Wasserstoff, Halogen, Niederalkyl, Niederalkoxy, $C_1$-$C_4$-Acylamino oder Phenyl,
$T_1$ und $T_2$ je Wasserstoff, Halogen, Hydroxy, Niederalkyl oder Niederalkoxy und
$V_1$, $V_2$, $V_3$ und $V_4$ je Wasserstoff, Niederalkyl, Cycloalkyl oder Benzyl oder
($V_1$ und $V_2$) oder ($V_3$ und $V_4$) je zusammen Alkylen bedeuten.

9. Chinazolinverbindungen gemäss Anspruch 8, dadurch gekennzeichnet, dass in Formel (3) $Y_1$ den Tetrahydrochinolinrest der Formel (3c) darstellt.

10. Chinazolinverbindungen gemäss Anspruch 9, dadurch gekennzeichnet, dass sie der Formel

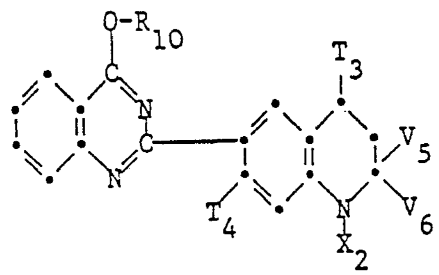

(4)

entsprechen, worin
$R_{10}$ Niederalkyl, Benzyl oder Phenyl,
$X_2$ Alkyl mit 1 bis 8 Kohlenstoffatomen, β-Cyanoethyl oder Benzyl,
$T_3$, $V_5$ und $V_6$ je Niederalkyl und
$T_4$ Wasserstoff oder Methyl bedeuten.

11. Verfahren zur Herstellung von Chinazolinverbindungen der im Anspruch 1 angegebenen Formel (1), in der Z —$OR_1$, —$SR_1$ oder —$NR_2R_3$ bedeutet, dadurch gekennzeichnet, dass man eine 4-Halogenchinazolinverbindung der Formel

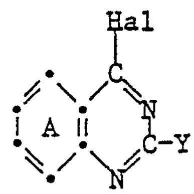

(5)

worin A und Y die im Anspruch 1 angegebene Bedeutung haben und Hal Halogen bedeutet mit einer Verbindung der Formel

$R_1$-OH , $R_1$-SH oder 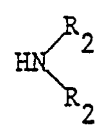

(6a) (6b) (6c)

umsetzt, worin $R_1$, $R_2$ und $R_3$ die im Anspruch 1 angegebene Bedeutung haben.

12. Verfahren zur Herstellung von Chinazolinverbindungen der im Anspruch 1 angegebenen Formel (1), in der Z Wasserstoff bedeutet, dadurch gekennzeichnet, dass man eine 4-Halogenchinazolinverbindung der Formel

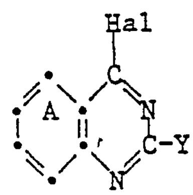

(5)

worin A und Y die im Anspruch 1 angegebene Bedeutung haben und Hal Halogen bedeutet, alkalisch enthalogeniert.

13. Verfahren zur Herstellung von Chinazolinverbindungen der in Anspruch 1 angegebenen Formel (1), in der Z den Rest R bedeutet, dadurch gekennzeichnet, dass man eine Ketoamidoverbindung der Formel

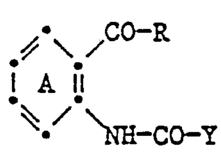

(11)

worin A, R und Y die im Anspruch 1 angegebene Bedeutung haben, mit einer alkoholischen Ammoniaklösung reagieren lässt.

14. Verwendung einer Chinazolinverbindung der in einem der Ansprüche 1 bis 10 angegebenen Formel als Farbbildner in einem druckempfindlichen oder wärmeempfindlichen Aufzeichnungsmaterial.

15. Druckempfindliches Aufzeichnungsmaterial, dadurch gekennzeichnet, dass es in seinem Farbreaktantensystem als Farbbildner mindestens eine Chinazolinverbindung der in einem der Ansprüche 1 bis 10 angegebenen Formel und mindestens einen festen Elektronenakzeptor enthält, wobei die Chinazolinverbindung gelöst in einem organischen Lösungsmittel und in Mikrokapseln eingekapselt ist.

16. Druckempfindliches Aufzeichnungsmaterial gemäss Anspruch 15, dadurch gekennzeichnet, dass die eingekapselte Chinazolinverbindung in Form einer Schicht auf der Rückseite eines Uebertragungsblattes und der Elektronenakzeptor in Form einer Schicht auf der Vorderseite des Empfangsblattes vorhanden sind.

17. Druckempfindliches Aufzeichnungsmaterial gemäss einem der Ansprüche 15 und 16, dadurch gekennzeichnet, dass die Chinazolinverbindung gemeinsam mit einem oder mehreren anderen Farbbildern enthalten ist.

18. Wärmeempfindliches Aufzeichnungsmaterial, dadurch gekennzeichnet, dass es in mindestens einer Schicht mindestens eine Chinazolinverbindung der in einem der Ansprüche 1 bis 10 angegebene Formel, einen Elektronenakzeptor und gegebenenfalls ein Bindemittel enthält.

19. Druckempfindliches Aufzeichnungsmaterial gemäss einem der Ansprüche 15 bis 18, dadurch gekennzeichnet, dass die Chinazolinverbindung gemeinsam mit einem oder mehreren anderen Farbbildern enthalten ist.

20. Wärmeempfindliches Aufzeichnungsmaterial gemäss Anspruch 15, dadurch gekennzeichnet, dass es in mindestens einer Schicht mindestens eine Chinazolinverbindung der in einem der Ansprüche 1 bis 10 angegebene Formel, einen Elektronenakzeptor und gegebenenfalls ein Bindemittel enthält.

**Claims**

1. A chromogenic quinazoline of the formula

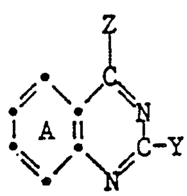

(1)

wherein

Y is the radical of a hydrogenated N-heterocyclic compound which is attached to the quinazoline moiety through a fused benzene nucleus present in said compound and is unsubstituted or substituted, and

Z is hydrogen, R, $-OR_1$, $-SR_1$ or $-NR_2R_3$, wherein

R and $R_1$ are each alkyl which contains not more than 12 carbon atoms and is unsubstituted or substituted by cyano or lower alkoxy, or are cycloalkyl, or phenyl, naphthyl, diphenyl, benzyl or phenylethyl, each of which is unsubstituted or substituted by halogen, cyano, nitro, lower alkyl, lower alkoxy, lower alkoxycarbonyl or lower alkylcarbonyl, or a 5- or 6-membered heterocyclic radical of aromatic character which is unsubstituted or substituted by halogen, cyano, lower alkyl, lower alkoxy or lower alkoxycarbonyl, and $R_1$ is also $C_2$-$C_6$ haloalkyl,

$R_2$ and $R_3$, each independently of the other, are hydrogen, alkyl which contains not more than 12 carbon atoms and is unsubstituted or substituted by halogen, hydroxy, cyano or lower alkoxy, or are cycloalkyl, phenyl, benzyl, or phenyl or benzyl which are each substituted by halogen, nitro, cyano, lower alkyl, lower alkoxy or lower alkoxycarbonyl, and $R_2$ is also $C_1$-$C_{12}$ acyl ; or

$R_2$ and $R_3$, together with the nitrogen atom to which they are attached, are a 5- or 6-membered, saturated heterocyclic radical, and the ring

A is unsubstituted or substituted by halogen, cyano, nitro, lower alkyl, lower alkoxy or lower alkoxycarbonyl, with the groups qualified by the term « lower » containing 1 to 5 carbon atoms.

2. A quinazoline according to claim 1, wherein Z is $-OR_1$.

3. A quinazoline according to either of claims 1 or 2, wherein Y is an indoline, tetrahydroquinoline or benzomorpholine radical.

4. A quinazoline according to claim 1, of the formula

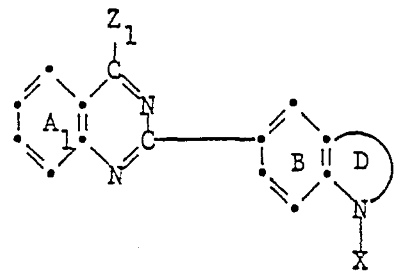

(2)

wherein

X is hydrogen, alkyl which contains not more than 8 carbon atoms and is unsubstituted or substituted by halogen, cyano or lower alkoxy, or is cycloalkyl or benzyl,

$Z_1$ is hydrogen, $R_4$, $—OR_4$, $—SR_4$ or $—NR_5R_6$, wherein

$R_4$ is alkyl which contains not more than 8 carbon atoms and is unsubstituted or substituted by lower alkoxy, or is cyclohexyl, phenyl, naphthyl, benzyl, or phenyl or benzyl which are each substituted by halogen, cyano, lower alkyl, lower alkoxy or lower alkoxycarbonyl,

$R_5$ and $R_6$, each independently of the other, are hydrogen, lower alkyl, phenyl, lower alkylphenyl, lower alkoxyphenyl or benzyl, and $R_5$ is also lower alkylcarbonyl, lower alkylsulfonyl, benzoyl or phenylsulfonyl, or

$R_5$ and $R_6$ together with the nitrogen atom to which they are attached are pyrrolidono, piperidino or morpholino, and the rings

$A_1$ and B, each independently of the other, are unsubstituted or substituted by cyano, halogen, lower alkyl, or lower alkoxy, and the ring

D is a hydrogenated 5- or 6-membered N-heterocyclic ring which may contain as ring member a further hetero atom and which is unsubstituted or C-substituted by one or more than one of the same or different substituents selected from the group consisting of halogen, cyano, hydroxyl, lower alkyl, lower alkoxy, $C_5$-$C_6$ cyloalkyl, benzyl and $C_3$-$C_6$ alkylene.

5. A quinazoline according to claim 4, wherein $Z_1$ is $—OR_4$ and $R_4$ is lower alkyl, benzyl or phenyl.

6. A quinazoline according to either of claims 4 or 5, wherein the ring D is 6-membered.

7. A quinazoline according to any one of claims 4 to 6, wherein X is lower alkyl, benzyl or β-cyanoethyl.

8. A quinazoline according to claim 1, of the formula

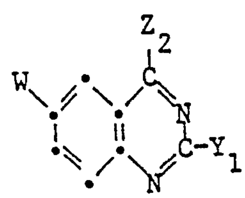

(3)

wherein

$Z_2$ is hydrogen, $R_7$, $—OR_7$, $—SR_7$ or $—NR_8R_9$,

$R_7$ is lower alkyl, lower alkoxy-lower alkyl, cyclohexyl, phenyl, naphthyl, benzyl, or phenyl which is substituted by halogen, cyano, methyl or methoxy,

$R_8$ and $R_9$, each independently of the other, are hydrogen, lower alkyl, phenyl or benzyl, and

$R_8$ is also lower alkylcarbonyl or benzoyl,

W is hydrogen, halogen, methyl or methoxy, and

$Y_1$ is a 5-indolinyl radical of the formula

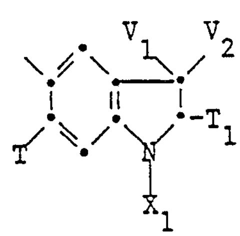

(3a)

a tetrahydroquinolinyl radical of the formula

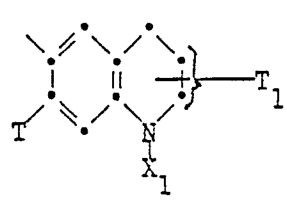

(3b)

a tetrahydroquinolinyl radical of the formula

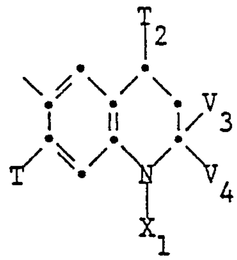

(3c)

or a benzomorpholino radical of the formula

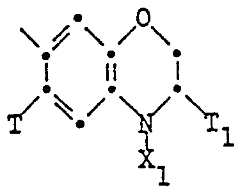

(3d)

$X_1$ is hydrogen, $C_1$-$C_8$ alkyl, $C_2$-$C_6$ alkoxyalkyl, β-cyanoethyl or benzyl,
T is hydrogen, halogen, lower alkyl, lower alkoxy, $C_1$-$C_4$ acylamino or phenyl,
$T_1$ and $T_2$ are each hydrogen, halogen, hydroxy, lower alkyl or lower alkoxy, and
$V_1$, $V_2$, $V_3$ and $V_4$ are each hydrogen, lower alkyl, cycloalkyl or benzyl, or
($V_1$ and $V_2$) or ($V_3$ and $V_4$) are each together alkylene.

9. A quinazoline according to claim 8, wherein $Y_1$ is the tetrahydroquinoline radical of the formula (3c).

10. A quinazoline according to claim 9, of the formula

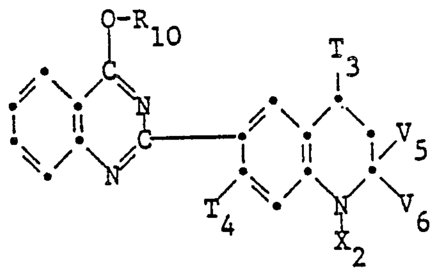

(4)

wherein
$R_{10}$ is lower alkyl, benzyl or phenyl,
$X_2$ is $C_1$-$C_8$ alkyl, β-cyanoethyl or benzyl,
$T_3$, $V_5$ and $V_6$ are each lower alkyl, and
$T_4$ is hydrogen or methyl.

11. A process for the preparation of a quinazoline of the formula (1) as indicated in claim 1, wherein Z is $—OR_1$, $—SR_1$ or $—NR_2R_3$, which process comprises reacting a 4-haloquinazoline of the formula

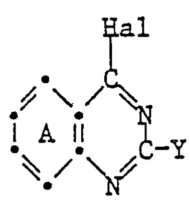

(5)

wherein A and Y are as defined in claim 1 and Hal is halogen, with a compound of the formula

$R_1$-OH (6a), $R_1$-SH (6b) or 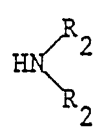 (6c).

in which formulae $R_1$, $R_2$ and $R_3$ are as defined in claim 1.

12. A process for the preparation of a quinazoline of the formula (1) as indicated in claim 1, wherein Z is hydrogen, which process comprises dehalogenating, under alkaline conditions, a 4-haloquinazoline of the formula

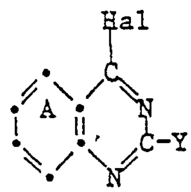

(5)

wherein A and Y are as defined in claim 1 and Hal is halogen.

13. A process for the preparation of a quinazoline of the formula (1) as indicated in claim 1, wherein Z is the radical R, which process comprises reacting a ketoamido compound of the formula

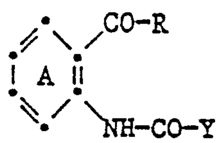

(11)

wherein A, R and Y are as defined in claim 1, with a solution of ammonia in alcohol.

14. Use of a quinazoline of the formula as indicated in any one of claims 1 to 10 as color former in a pressure-sensitive or heat-sensitive recording material.

15. A pressure-sensitive recording material which comprises a support which contains, or has coated thereon as color former, at least one quinazoline of the formula as indicated in any one of claims 1 to 10 and at least one solid electron acceptor, said quinazoline being dissolved in an organic solvent and encapsulated in microcapsules.

16. The pressure-sensitive recording material of claim 15, wherein the encapsulated quinazoline is present in the form of a layer on the back of a transfer sheet and the electron acceptor is present in the form of a layer on the face of the receiving sheet.

17. The pressure-sensitive recording material of either of claims 15 or 16, which comprises the quinazoline together with one or more other color formers.

18. A heat-sensitive recording material, which comprises in at least one layer, at least one quinazoline of the formula as indicated in any one of claims 1 to 10, at least one electron acceptor and at least one binder.

19. The heat-sensitive recording material of claim 18, which comprises the quinazoline together with one or more other color formers.

20. The heat-sensitive recording material of either of claims 18 or 19, which comprises in at least one layer, at least one quinazoline of the formula as indicated in any one of claims 1 to 10, at least one electron acceptor and at least one binder.

**Revendications**

1. Composés quinazoliniques chromogènes de formule

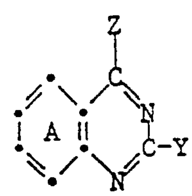

(1)

dans laquelle

Y représente le reste d'un composé hétérocyclique azoté hydrogéné, qui est lié à la partie quinazolinique par un noyau benzénique condensé qui y est inclus, et qui est non substitué ou substitué, et

Z représente un hydrogène, R, $—OR_1$, $—SR_1$ ou $—NR_2R_3$, où

R et $R_1$ représentent chacun un reste alkyle ayant au maximum 12 atomes de carbone, non substitué ou à substituant(s) cyano ou alcoxy inférieur, un reste cycloalkyle, le reste phényle, le reste naphtyle, le reste diphényle, le reste benzyle ou le reste phényléthyle, ces restes étant à chaque fois non substitués ou à substituant(s) halogène, cyano, nitro, alkyle inférieur, alcoxy inférieur, (alcoxy inférieur)-carbonyle ou

(alkyle inférieur)-carbonyle, un reste hétérocyclique de 5 ou 6 chaînons de caractère aromatique, qui est non substitué ou à substituant(s) halogène, cyano, alkyle inférieur, alcoxy inférieur ou (alcoxy inférieur)-carbonyle et $R_1$ représente aussi un reste halogénoalkyle ayant 2 à 6 atomes de carbone,

$R_2$ et $R_3$ représentent, indépendamment l'un de l'autre, un hydrogène, un reste alkyle ayant au maximum 12 atomes de carbone, non substitué ou à substituant(s) halogène, hydroxy, cyano ou alcoxy inférieur, ou bien un reste cycloalkyle, le reste phényle, le reste benzyle, ou un reste phényle ou benzyle à substituant(s) halogène, eitro, cyano, alkyle inférieur, alcoxy inférieur ou (alcoxy inférieur)-carbonyle, et $R_2$ représente aussi un reste acyle ayant de 1 à 12 atomes de carbone, ou bien

$R_2$ et $R_3$ représentent ensemble, avec l'atome d'azote auquel ils sont liés, un reste hétérocyclique saturé à 5 ou 6 chaînons, et le cycle

A n'est pas substitué ou est à substituant(s) halogène, cyano, nitro, alkyle inférieur, alcoxy inférieur ou (alcoxy inférieur)-carbonyle, l'expression « inférieur » se rapportant à des groupes ayant de 1 à 5 atomes de carbone.

2. Composés quinazoliniques selon la revendication 1, caractérisés par le fait que, dans la formule (1), Z représente le reste $—OR_1$.

3. Composés quinazoliniques selon une des revendications 1 et 2, caractérisés par le fait que, dans la formule (1), Y représente un reste indolinique, tétrahydroquinolinique ou benzomorpholinique.

4. Composés quinazoliniques selon la revendication 1, caractérisés par le fait qu'ils correspondent à la formule

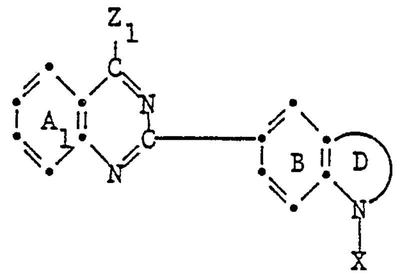

(2)

dans laquelle

X représente un hydrogène ou un reste alkyle ayant au maximum 8 atomes de carbone, non substitué ou à substituant(s) halogène, cyano ou alcoxy inférieur, un reste cycloalkyle ou le reste benzyle et

$Z_1$ représente un hydrogène, $R_4$, $—OR_4$, $—SR_4$ ou $NR_5R_6$, où

$R_4$ représente un reste alkyle ayant au maximum 8 atomes de carbone, non substitué ou à substituant(s) alcoxy inférieur, le reste cyclohexyle, le reste phényle, le reste naphtyle, le reste benzyle ou un reste phényle ou benzyle à substituant(s) halogène, cyano, alkyle inférieur, alcoxy inférieur ou (alcoxy inférieur)-carbonyle,

$R_5$ et $R_6$ représentent, indépendammment l'un de l'autre, un hydrogène, un reste alkyle inférieur, le reste phényle, un reste (alkyle inférieur)-phényle, un reste (alcoxy inférieur)-phényle ou le reste benzyle et $R_5$ représente aussi un reste (alkyle inférieur)-carbonyle, un reste (alkyle inférieur)-sulfonyle, le reste benzoyle ou le reste phénylsulfonyle ou bien

$R_5$ et $R_6$ représentent ensemble, avec l'atome d'azote auquel ils sont liés, un groupe pyrrolidino, pipéridino ou morpholino, et où les cycles

$A_1$ et B, indépendamment l'un de l'autre, ne sont pas substitués ou sont à substituant(s) cyano, halogène, alkyle inférieur ou alcoxy inférieur, et le cycle

D représente un hétérocycle azoté hydrogéné à 5 ou 6 chaînons, qui présente éventuellement comme membre du cycle un autre hétéroatome et qui est non substitué ou substitué sur les carbones, une fois ou plusieurs fois, par des groupes cyano, hydroxyle, alkyle inférieur, alcoxy inférieur, cycloalkyle en $C_5$-$C_6$, benzyle ou alkylène en $C_3$-$C_6$, ou des atomes d'halogène.

5. Composés quinazoliniques selon la revendication 4, caractérisés par le fait que, dans la formule (2), $Z_1$ représente le reste $—OR_4$ et $R_4$ représente un reste alkyle inférieur, le reste benzyle ou le reste phényle.

6. Composés quinazoliniques selon une des revendications 4 et 5, caractérisés par le fait que, dans la formule (2), le cycle D est à six chaînons.

7. Composés quinazoliniques selon une des revendications 4 à 6, caractérisés par le fait que, dans la formule (2), X représente un reste alkyle inférieur, le reste benzyle ou le reste β-cyanoéthyle.

8. Composés quinazoliniques selon la revendication 1, caractérisés par le fait qu'ils correspondent à la formule

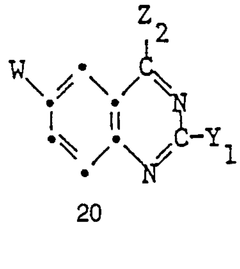

(3)

dans laquelle

$Z_2$ représente un hydrogène, $R_7$, $—OR_7$, $SR_7$ ou $NR_8R_9$,

$R_7$ représente un reste alkyle inférieur, un reste (alcoxy inférieur)-alkyle inférieur, le reste cyclohexyle, le reste phényle, le reste naphtyle, le reste benzyle ou un reste phényle à substituant(s) halogène, cyano, méthyle ou méthoxy,

$R_8$ et $R_9$ représentent, indépendamment l'un de l'autre, un hydrogène, un reste alkyle inférieur, le reste phényle ou le reste benzyle et

$R_8$ représente aussi un reste (alkyle inférieur)-carbonyle ou le reste benzoyle,

W représente un hydrogène, un halogène, le reste méthyle ou le reste méthoxy et

$Y_1$ représente un reste 5-indolinyle de formule

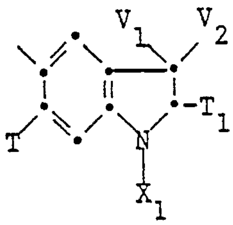

(3a)

un reste tétrahydroquinolinylique de formule

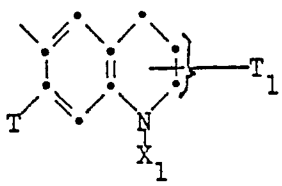

(3b)

un reste tétrahydroquinolinylique de formule

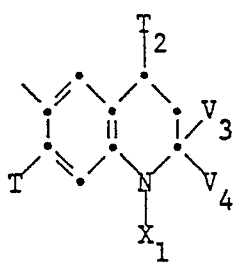

(3c)

ou un reste benzomorpholino de formule

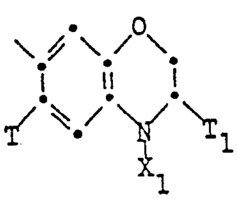

(3d)

$X_1$ représente un hydrogène, un reste alkyle en $C_1$-$C_8$, un reste alcoxyalkyle en $C_2$-$C_6$, le reste β-cyanoéthyle, le reste benzyle,

T représente un hydrogène, un halogène, un reste alkyle inférieur, un reste alcoxy inférieur, un groupe (acyle en $C_1$-$C_4$)-amino ou le reste phényle,

$T_1$ et $T_2$ représentent chacun un hydrogène, un halogène, un groupe hydroxy, un reste alkyle inférieur ou un reste alcoxy inférieur et

$V_1$, $V_2$, $V_3$ et $V_4$ représentent chacun un hydrogène, un reste alkyle inférieur, un reste cycloalkyle ou le reste benzyle ou bien

($V_1$ et $V_2$) ou ($V_3$ et$V_4$) représentent ensemble un reste alkylène.

9. Composés quinazoliniques selon la revendication 8, caractérisés par le fait que, dans la formule (3), $Y_1$ représente le reste tétrahydroquinolinique de formule (3c).

10. Composés quinazoliniques selon la revendication 9, caractérisés par le fait qu'ils correspondent à la formule

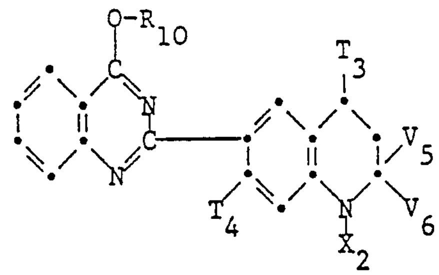

(4)

dans laquelle

$R_{10}$ représente un reste alkyle inférieur, le reste benzyle ou le reste phényle,

$X_2$ représente un reste alkyle ayant de 1 à 8 atomes de carbone, le reste β-cyanoéthyle ou le reste benzyle,

$T_3$, $V_5$ et $V_6$ représentent chacun un reste alkyle inférieur et

$T_4$ représente un hydrogène ou le reste méthyle.

11. Procédé de préparation des composés quinazoliniques ayant la formule (1) indiquée dans la revendication (1), dans laquelle Z représente —$OR_1$, —$SR_1$ ou —$NR_2R_3$, caractérisé par le fait que l'on fait réagir un composé 4-halogénoquinazolinique de formule

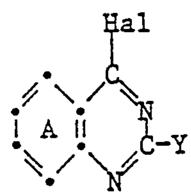

(5)

dans laquelle A et Y ont la signification indiquée dans la revendication 1 et Hal représente un halogène, avec un composé de formule

$R_1$-OH , $R_1$-SH ou 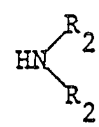

(6a) (6b) (6c)

où $R_1$, $R_2$ et $R_3$ ont la signification indiquée dans la revendication 1.

12. Procédé de préparation des composés quinazoliniques ayant la formule (1) indiquée dans la revendication 1, dans laquelle Z représente un hydrogène, caractérisé par le fait que l'on deshalogène par un alcalin un composé 4-halogénoquinazolinique de formule

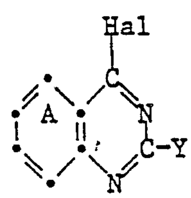

(5)

où A et Y ont la signification indiquée dans la revendication 1 et Hal représente un halogène.

13. Procédé de préparation des composés quinazoliniques ayant la formule (1) indiquée dans la revendication 1, dans laquelle Z représente le reste R, caractérisé par le fait que l'on fait réagir avec une solution alcoolique d'ammoniac un composé cétoamide de formule

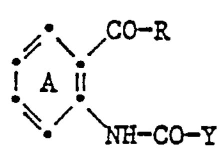

(11)

où A, R et Y ont la signification indiquée dans la revendication 1.

14. Utilisation d'un composé quinazolinique ayant la formule indiquée dans une des revendications 1 à 10 comme chromogène dans un matériau d'enregistrement sensible à la pression ou sensible à la chaleur.

15. Matériau d'enregistrement sensible à la pression, caractérisé par le fait qu'il contient dans son système de réactifs de coloration, comme chromogène, au moins un composé quinazolinique ayant la formule indiquée dans une des revendications 1 à 10 et au moins un accepteur d'électrons solide, le composé quinazolinique étant dissous dans un solvant organique et étant encapsulé dans des microcapsules.

16. Matériau d'enregistrement sensible à la pression selon la revendication 15, caractérisé par le fait que le composé quinazolinique encapsulé est présent sous la forme d'une couche sur le côté verso d'une feuille de transfert, et l'accepteur d'électrons est présent sous la forme d'une couche sur le côté recto d'une feuille de réception.

17. Matériau d'enregistrement sensible à la pression selon une des revendications 15 et 16, caractérisé par le fait que le composé quinazolinique est contenu simultanément avec un ou plusieurs autres chromogènes.

18. Matériau d'enregistrement sensible à la chaleur, caractérisé par le fait qu'il contient, dans au moins une couche, au moins un composé quinazolinique ayant la formule indiquée dans une des revendications 1 à 10, un accepteur d'électrons et éventuellement un liant.

19. Matériau d'enregistrement sensible à la chaleur selon la revendication 18, caractérisé par le fait que le composé quinazolinique est contenu simultanément avec un ou plusieurs autres chromogènes.

20. Matériau d'enregistrement sensible à la chaleur selon une des revendications 18 et 19, caractérisé par le fait qu'il contient, dans au moins une couche, au moins un composé quinazolinique ayant la formule indiquée dans une des revendications 1 à 10, un accepteur d'électrons et éventuellement un liant.